# EUROPEAN PATENT APPLICATION

(11) **EP 4 744 896 A1**
(43) Date of publication of application: **20.05.2026**
(21) Application number: 24315522.3
(22) Date of filing: 15.11.2024
(51) Int. Cl.: B33Y 70/00, A61L 24/04, C08G 63/47, C08G 63/685, C08G 63/91

(54) **COMPOSITION COMPRISING PRE-POLYMER**

(71) Applicant: Tissium SA, 75012 Paris (FR)
(72) Inventor: Thomas, Emmanuel, 93500 PANTIN (FR); Balarezo, Mauricio, 92220 BAGNEUX (FR); Suty, Benjamin, 93160 NOISY LE GRAND (FR)
(74) Representative: Opilex

(57) **Abstract**

A composition comprising a pre-polymer having a polymeric backbone comprising a polymeric unit of the formula (-A-B-)ₙ, wherein A is derived from a substituted or unsubstituted polyol or mixture thereof and B is derived from a substituted or unsubstituted polyacid or mixture thereof, and n is an integer greater than 1, is disclosed. The composition may be used in a method of fixing to or sealing tissue, for fixing a medical device to the surface of a tissue, or for preparing a printing resin.

## Description

### FIELD OF INVENTION

The present invention relates to a composition comprising a pre-polymer, a method of manufacturing the composition, a method of curing the composition, a cured composition obtainable therefrom, uses of the composition and methods of using the composition.

### BACKGROUND OF THE INVENTION

Polymeric tissue sealants and adhesives provide materials for tissue repair and are widely used in a variety of medical settings ranging from minor to life-threatening tissue injuries. They are, for example, an alternative to sutures and staples to close and seal wounds or incisions. These materials are advantageous because of their ease of use, short application time and minimal tissue damage, making them suitable for minimally invasive procedures. An ideal tissue adhesive should have the following properties: strong adhesion to tissue, especially under wet conditions; an ability to form a watertight seal; biodegradability; mechanical compliance with the underlying substrate (that is, the tissue); low cytotoxicity and minimal inflammatory response. Despite their numerous current applications, tissue adhesives still face several limitations and unresolved challenges (e.g., weak adhesion strength, poor mechanical properties and safety issues) that limit their use, leaving ample room for improvements.

Current clinically-available adhesives, such as medical grade cyanoacrylate (CA) or fibrin sealant, are easily washed out or cured under dynamic wet conditions, are toxic and cannot be used internally, and/or exhibit weak adhesive properties such that they cannot withstand the forces inside the cardiac chambers and major blood vessels. Also, many of these adhesives exhibit activation properties that make fine adjustments or repositioning of the devices very difficult. Moreover, many adhesives under development achieve tissue adhesion only through chemical reaction with functional groups at the tissue surface, and thus become ineffective in the presence of blood.

Alternatives to cyanoacrylate have been explored. US 8,143,042 describes biodegradable elastomers prepared by crosslinking a pre-polymer containing crosslinkable functional groups, such as acrylate groups. It also discloses that it is desirable to increase the number of free hydroxy groups on the polymer in order to increase the stickiness of the polymer. Increasing the number of hydroxy groups in the backbone also leads to enhanced solubility in physiologic solutions. This suggests that the primary mechanism of adhesion of the polymer is chemical interactions between functional groups, for example free hydroxy groups on the polymer and the tissue to which it is applied. However, this type of chemical interaction becomes ineffective in the presence of body fluids, especially blood, as shown in Artzi et al., Adv. Mater. 21, 3399-3403 (2009).

Elastomeric crosslinked polyesters are disclosed in US 2013/0231412. Biodegradable polymers are disclosed in US 7,722,894. Adhesive articles are disclosed in WO2009/067482 and WO2014/190302. Blood resistant surgical glue is described in Lang et al. "A Blood-Resistant Surgical Glue for Minimally Invasive Repair of Vessels and Heart Defects," Sci. Transl. Med., 8 January 2014: Vol. 6, Issue 218, p. 218ra6 and WO2014/190302.

WO 2021/078962 discloses polyglycerol sebacate (PGS) polymers that have been acrylated by reaction with acryloyl chloride and a proportion of the acrylated moities are then aminated by reaction with diethylamine. The acrylated and aminated pre-polymer is acidified to provide positively charged nitrogen groups. An improvement in adhesion is observed as the zeta potential of the pre-polymer compositions increases.

### SUMMARY OF THE INVENTION

The invention provides an improved and commercially viable pre-polymer that can be readily applied to the desired site, is biocompatible (non-toxic), and exhibits strong forces once cured/crosslinked leading to improved tissue sealant/fixation.

The pre-polymer remains in place at the desired site prior to curing/crosslinking, even in the presence of body fluids, such as blood.

The pre-polymer exhibits improved stability over time compared to previously disclosed pre-polymers. More particularly, the pre-polymer exhibits improved stability over time at room temperature and up to 45°C.

More particularly, the invention provides a composition comprising a pre-polymer having a polymeric backbone comprising a polymeric unit of the formula (-A-B-)ₙ, wherein A is derived from a substituted or unsubstituted polyol or mixture thereof and B is derived from a substituted or unsubstituted polyacid or mixture thereof, and n is an integer greater than 1,
wherein the pre-polymer has activated groups on its polymeric backbone,
and wherein the pre-polymer further comprises groups of formula (I): 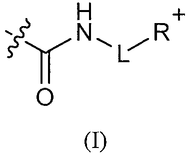
wherein L is a linking moiety, and wherein R⁺ is a moiety comprising a positively charged heteroatom.

The present invention also provides methods for preparing the pre-polymer and composition of the present invention.

The present invention further provides a method of curing the composition according to the present invention, comprising curing the composition with a stimulus, for example light in the presence of a photo-initiator.

The present invention also provides a cured composition obtainable by the curing method according to the present invention. Desirably said cured composition can fix strongly to a surface or can fix one surface to another.

The present invention further provides methods of use and use of the composition according to the present invention for sealing tissue or for fixing a medical device to the surface of tissue.

The present invention further provides methods of use and use of the composition according to the present invention for preparing printing resin (e.g. 3D printing resin) and for producing shaped objects by a variety of techniques known in the art, including 3D printing. The invention further provides printed (e.g. 3D printed) shaped objects obtained by using the composition of the invention.

The inventors have found that, compared to known compositions, the present invention offers advantages which are not found in the prior art.

### BRIEF DESCRIPTION OF THE FIGURES

Figure 1 shows a scheme of the synthesis of a comparative pre-polymer.
Figure 2 shows a scheme of the synthesis of a pre-polymer comprised in a composition according to the invention.
Figure 3 shows a graph indicating how the amount of amide groups varies with the reaction conditions.
Figure 4 shows a graph indicating how the amount of acrylate groups varies with the reaction conditions.
Figure 5 shows a graph of the results of accelerated stability assessment for compositions according to the invention and comparative compositions.

### DETAILED DESCRIPTION OF THE INVENTION

### Pre-polymer

The polymeric backbone of the pre-polymer comprises a polymeric unit of the general formula (-A-B-)ₙ, wherein A is derived from a substituted or unsubstituted polyol or mixture thereof and B is derived from a substituted or unsubstituted polyacid or mixture thereof; and n represents an integer greater than 1. The polymeric backbone is made up of repeating monomer units of general formula -A-B-.

The term "substituted" has its usual meaning in chemical nomenclature and is used to describe a chemical compound in which a hydrogen on the primary carbon chain has been replaced with a substituent such as alkyl, aryl, carboxylic acid, ester, amide, amine, urethane, ether, or carbonyl.

Component A of the pre-polymer is derived from a polyol or mixture thereof, such as a diol, triol, tetraol or greater. Suitable polyols include diols, such as alkane diols, e.g. octanediol; triols, such as glycerol, trimethylolpropane, trimethylolpropane ethoxylate, triethanolamine; tetraols, such as erythritol, pentaerythritol; and higher polyols, such as sorbitol. Component A may also be derived from unsaturated polyols, such as tetradeca-2,12-diene-1,14-diol, polybutadiene-diol or other polyols including macromonomer polyols such as, for example polyethylene oxide, polycaprolactone triol and N-methyldiethanoamine (MDEA) can also be used. In one embodiment, the polyol is substituted or unsubstituted glycerol.

In an embodiment of the invention where the polyol is a diol, the diol may be a polyether. A polyether is a polymer wherein the repeating unit contains a carbon-oxygen bond. The polyether may be polyethylene glycol: 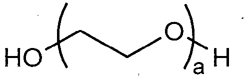 a is an integer and will be a range of different values for a polydisperse polyethylene glycol. The molecular weight (i.e. number average molecular weight, Mₙ) of the polyethylene glycol is preferably greater than 150 g/mol and more preferably greater than 200 g/mol. The molecular weight (i.e. number average molecular weight, Mₙ) of the polyethylene glycol is preferably less than 1500 g/mol, more preferably less than 1000 g/mol, even more preferably less than 700 g/mol. Commercial polyethylene glycol grades that would be suitable for use in the present invention include PEG200, PEG400 and PEG600.

In another embodiment of the invention where the polyol is a diol, the diol is an aliphatic diol wherein the hydroxy groups are on the terminal carbon atoms: 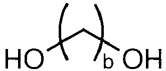 b is an integer of 4 or more, preferably an integer of 6 or more. Diols that could be used include 1,8-octanediol; 1,7-heptanediol; 1,6-hexanediol; 1,5-pentanediol; and 1,4-butanediol.

In another embodiment of the invention where the polyol is a diol, the diol is an aliphatic diol wherein the hydroxy groups are not on the terminal carbon atoms. A commercial aliphatic diol that would be suitable for use in the present invention is 2-butyl-2-ethyl-1,3-propanediol.

In another embodiment of the invention where the polyol is a diol, the diol is polycaprolactone diol.

Component B of the pre-polymer is derived from a polyacid or mixture thereof, preferably diacid or triacid. Exemplary acids include, but are not limited to, succinic acid (4 carbons), glutaric acid (5 carbons), adipic acid (6 carbons), pimelic acid (7 carbons), suberic acid (8 carbons), azelaic acid (9 carbons), sebacic acid (10 carbons), and citric acid. Exemplary long chain diacids include diacids having more than 10, more than 15, more than 20, and more than 25 carbon atoms. Polyethylene glycol diacid, e.g. PEG 600 diacid, could be used. Non-aliphatic diacids can also be used. For example, versions of the above diacids having one or more double bonds can be used to produce polyol-diacid co-polymers. Preferably the polyacid is substituted or unsubstituted sebacic acid.

Polyol-based polymers described in US 2011/0008277, US 7,722,894 and US 8,143,042, the contents of which are hereby incorporated by reference, are suitable polymeric backbones for use in the present invention.

Several substituents, such as amines, amides, urethanes, aldehydes, hydrazides, acrylates and aromatic groups, can be incorporated into the carbon chain. Exemplary aromatic diacids include terephthalic acid and carboxyphenoxy-propane. The diacids can also include substituents. For example, reactive groups like amine and hydroxy can be used to increase the number of sites available for cross-linking. Amino acids and other biomolecules can be used to modify the biological properties. Aromatic groups, aliphatic groups, and halogen atoms can be used to modify the inter-chain interactions within the polymer.

The weight average molecular weight of the pre-polymer (Mw), measured by Gel Permeation Chromatography equipped with a refractive index and polystyrene calibration standards, may be from about 1,000 Daltons to about 1,000,000 Daltons, preferably from about 2,000 Daltons to about 500,000 Daltons, more preferably from about 2,000 Daltons to about 250,000 Daltons, most preferably from about 2,000 Daltons to about 100,000 Daltons. The weight average molecular weight may be less than about 100,000 Daltons, less than about 75,000 Daltons, less than about 50,000 Daltons, less than about 40,000 Daltons, less than about 30,000 Daltons, or less than about 20,000 Daltons. The weight average molecular weight may be from about 1,000 Daltons to about 10,000 Daltons, from about 2,000 Daltons to about 10,000 Daltons, from about 3000 Daltons to about 10,000 Daltons, from about 2,000 Daltons to about 5,000 Daltons.

The term "about" as used herein means within 10%, preferably within 8%, and more preferably within 5% of a given value or range. According to a specific embodiment, "about X" means X, when X refers to the value or range.

The pre-polymer may have a polydispersity, measured by Gel Permeation Chromatography equipped with a refractive index and polystyrene calibration standards, below 20.0, more preferably below 10.0, more preferably below 5.0, and even more preferably below 3.5.

The molar ratios of the polyol to the polyacid in the pre-polymer are suitably in the range of about 0.5:1 to about 1.5:1, preferably in the range of about 0.9:1.1 to about 1.1:0.9 and most preferably about 1:1.

### Groups of Formula (I)

The pre-polymer of the invention comprises groups of formula (I): 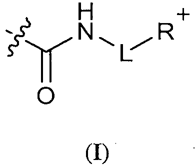 wherein L is a linking moiety, and wherein R⁺ is a moiety comprising a positively charged heteroatom.

The positively charged heteroatom in the R⁺ moiety may be derived from any element other than carbon or hydrogen. Preferred positively charged heteroatoms are nitrogen, phosphorus and sulfur. Most preferably, the positively charged heteroatom is a positively charged nitrogen atom.

In one embodiment the R⁺ moiety is an ammonium group preferably an ammonium group of formula -N⁺R^{a}R^{b}H wherein the positively charged nitrogen forms a bond with L, and R^{a} and R^{b} are independently chosen from C₁₋₂₀ alkyl groups, benzyl, substituted benzyl, cycloalkyl or substituted cycloalkyl, or R^{a} and R^{b} are linked such that R^{a} and R^{b} form a saturated cyclic group having 2 to 20 carbon atoms. In one embodiment, R^{a} and R^{b} are both C₁₋₆ alkyl, preferably ethyl. A cycloalkyl group is preferably a cyclopentyl, cyclohexyl, cycloheptyl or cyclooctyl group, optionally substituted with C₁₋₆ alkyl. A substituted benzyl group is suitably substituted with C₁₋₆ alkyl.

In another embodiment the R⁺ moiety is of formula -N⁺R^{a}R^{b}R^{c} wherein the positively charged nitrogen forms a bond with L, and R^{a}, R^{b} and R^{c} are independently chosen from C₁₋₂₀ alkyl groups, benzyl, substituted benzyl, cycloalkyl or substituted cycloalkyl, or at least two of R^{a}, R^{b} and R^{c} are linked such that R^{a}, R^{b} and/or R^{c} form a saturated cyclic group having 2 to 20 carbon atoms. A cycloalkyl group is preferably a cyclopentyl, cyclohexyl, cycloheptyl or cyclooctyl group, optionally substituted with C₁₋₆ alkyl. A substituted benzyl group is suitably substituted with C₁₋₆ alkyl.

In another embodiment the R⁺ moiety is a charged thiol, preferably of formula -S⁺R^{a}H wherein the positively charged sulfur forms a bond with L, and R^{a} is independently chosen from C₁₋₂₀ alkyl groups, benzyl, substituted benzyl, cycloalkyl or substituted cycloalkyl. In one embodiment, R^{a} is C₁₋₆ alkyl, preferably ethyl. A cycloalkyl group is preferably a cyclopentyl, cyclohexyl, cycloheptyl or cyclooctyl group, optionally substituted with C₁₋₆ alkyl. A substituted benzyl group is suitably substituted with C₁₋₆ alkyl.

In another embodiment the R⁺ moiety is a charged phosphine, preferably of formula -P⁺R^{a}R^{b}H wherein the positively charged phosphurs forms a bond with L, and R^{a} and R^{b} are independently chosen from C₁₋₂₀ alkyl groups, benzyl, substituted benzyl, cycloalkyl or substituted cycloalkyl, or R^{a} and R^{b} are linked such that R^{a} and R^{b} form a saturated cyclic group having 2 to 20 carbon atoms. In one embodiment, R^{a} and R^{b} are both C₁₋₆ alkyl, preferably ethyl. A cycloalkyl group is preferably a cyclopentyl, cyclohexyl, cycloheptyl or cyclooctyl group, optionally substituted with C₁₋₆ alkyl. A substituted benzyl group is suitably substituted with C₁₋₆ alkyl.

L is a linking moiety suitably chosen from a C₁₋₂₀ alkyl group, a C₂₋₂₀ alkenyl group, a C₂₋₂₀ alkynyl group, or a C₇₋₂₀ benzyl group wherein the groups are optionally substituted, and wherein the groups may optionally contain heteroatoms such as oxygen, nitrogen or sulfur. L is preferably a C₁₋₆ alkyl group. Alternatively, L may be an ethylene glycol chain, wherein the ethylene glycol chain is optionally substituted.

Preferred groups of formula (I) are shown below:

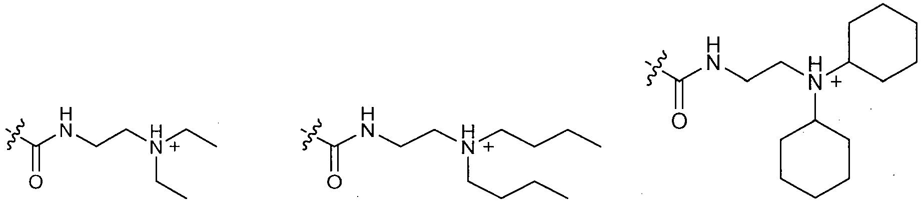

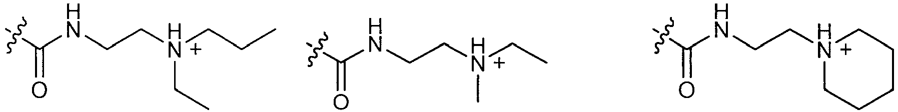

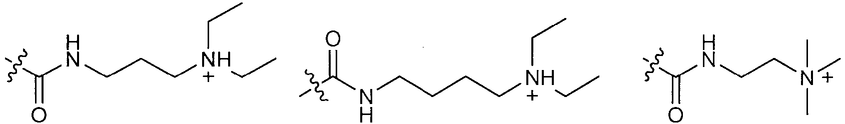

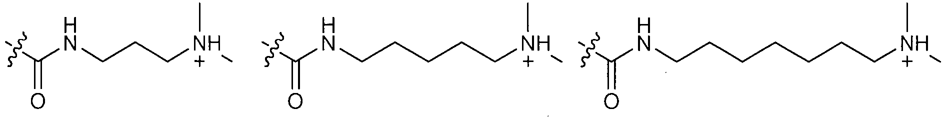

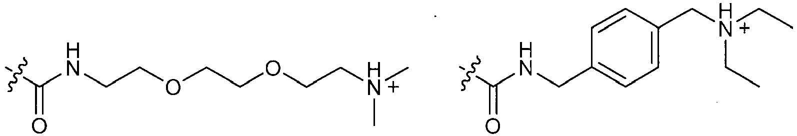

The proportion of groups of formula (I) compared to the number of monomer units in the backbone can vary depending on the polymer and may suitably range from about 0.05 to about 0.55 mol/mol of monomer unit. The proportion of groups of formula (I) is suitably measured by a technique such as ¹H NMR. The proportion of groups of formula (I) may be quoted per mole of polyacid or per mole of polyol. The ranges quoted above are preferably mol/mol of polyacid. When the groups of formula (I) include a positively charged nitrogen atom, then the proportion of groups of formula (I) is suitably characterized as "DN+". This is the number of positively charged nitrogen atoms compared to the number of monomer units in the backbone. The DN+ parameter is suitably determined using ¹H- NMR spectroscopy, using the characteristic peaks of hydrogen atoms adjacent to the positively charged nitrogen atom. The DN+ parameter is suitably quoted as mol/mol of polyacid.

The groups of formula (I) may be located anywhere on the pre-polymer. In one embodiment, the groups of formula (I) are located at terminal positions in the pre-polymer, i.e. on monomers that are at the end of polymer chains.

### Activated Groups

The pre-polymer in the composition of the invention has activated groups on its polymeric backbone.

The activated groups are functional groups that can react or be reacted to form crosslinks. The pre-polymer may be activated by reacting one or more functional groups on the monomer units of the backbone to provide one or more functional groups that can react or be reacted to form crosslinks resulting in cured polymer.

Suitable functional groups to be activated on the pre-polymer backbone include hydroxy groups, carboxylic acid groups, amines, and combinations thereof, preferably hydroxy and/or carboxylic acid. The free hydroxy or carboxylic acid groups on the pre-polymer can be activated by functionalizing the hydroxy groups with a moiety which can form a crosslink between polymer chains. The groups that are activated can be free hydroxy or carboxylic acid groups on A and/or B moieties in the pre-polymer.

The free hydroxy or carboxylic groups can be functionalized with a variety of functional groups, for example vinyl groups. Vinyl groups can be introduced by a variety of techniques known in the art, such as by vinylation or acrylation. According to the present invention, vinyl groups contain the following structure -CRₚ=CR_{q}Rᵣ wherein Rₚ, R_{q}, Rᵣ are independently from one another, selected from the group consisting of H, alkyl such as methyl or ethyl, aryl such as phenyl, substituted alkyl, substituted aryl, carboxylic acid, ester, amide, amine, urethane, ether, and carbonyl.

Preferably, the activated group is or contains an acrylate group. According to the present invention, acrylate groups may contain the following group: -C(=O)-CRₚ=CR_{q}Rᵣ, wherein Rₚ, R_{q}, Rᵣ are independently from one another, selected from the group consisting of H, alkyl such as methyl or ethyl, aryl such as phenyl, substituted alkyl, substituted aryl, carboxylic acid, ester, amide, amine, urethane, ether, and carbonyl. According to another embodiment, acrylate groups may contain the following group: -C(=O)NR_{d}-(CRₑR_{f})ₘ-O-C(=O)-CRₚ=CR_{q}Rᵣ, wherein Rₚ, R_{q}, Rᵣ, R_{d}, Rₑ and R_{f} are independently from one another, selected from the group consisting of H, alkyl, such as methyl or ethyl, aryl, such as phenyl, substituted alkyl, substituted aryl, carboxylic acid, ester , amide, amine, urethane, ether, and carbonyl; and m is an integer greater than 1 (e.g. m is 2). According to an embodiment, the activated pre-polymer contains a mixture of different acrylate groups.

Preferably, all or part of the acrylate groups containing the -C(=O)-CRₚ=CR_{q}Rᵣ group are such that Rₚ, R_{q} and Rᵣ are H; or such that Rₚ is CH₃, R_{q} and Rᵣ are H; or such that Rₚ and R_{q} are H and Rᵣ is CH₃; or such that Rₚ and R_{q} are H and Rᵣ is phenyl.

Preferably, all or part of the acrylate groups containing the -C(=O)NR_{d}-(CRₑR_{f})ₘ-O-C(=O)-CRₚ=CR_{q}Rᵣ group are such that R_{d}, Rₑ, R_{f}, Rₚ, R_{q} and Rᵣ are H, and m is 2; or such that Rₚ is CH₃, R_{d}, Rₑ, R_{f}, R_{q} and Rᵣ are H, and m is 2; or such that R_{d}, Rₑ, R_{f}, Rₚ and R_{q} are H, Rᵣ is CH₃, and m is 2; or such that R_{d}, Rₑ, R_{f}, Rₚ and R_{q} are H, Rᵣ is phenyl and m is 2.

The degree of activation (e.g. acrylation) is suitably measured by a technique such as ¹H NMR. The degree of activation (e.g. acrylation) is suitably characterized as "DA". The proportion of activated groups may be compared to the number of monomer units in the backbone. This can vary and can be from 0.1 to 0.8 mol/mol of monomer unit, preferably from 0.2 to 0.6 mol/mol of monomer unit. It is most preferred when the degree of activation is as described above and the reactive functional group is acrylate i.e. degree of acrylation as above. The proportion of activated groups may be quoted per mole of polyacid or per mole of polyol. The DA ranges quoted above are preferably mol/mol of polyacid.

In addition to acrylates or other vinyl groups, other agents can be used to provide activated groups on the pre-polymer backbone. Examples of such agents include, but are not limited to, glycidyl, epichlorohydrin, triphenylphosphine, diethyl azodicarboxylate (DEAD), diazirine, divinyladipate, and divinylsebacate with the use of enzymes as catalysts, phosgene-type reagents, di-acid chlorides, bis-anhydrides, bis-halides, metal surfaces, and combinations thereof. Agents may further include isocyanate, aldehyde, epoxy, vinyl ether, thiol, DOPA residues or N-Hydroxysuccinimide functional groups.

### Pre-polymer with groups of formula (I) and activated groups

In an embodiment of the invention, the pre-polymer is of formula (A): 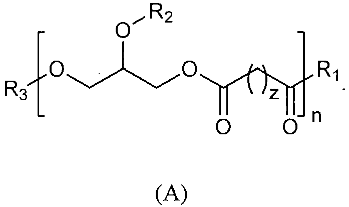
wherein the pre-polymer is branched such that there are multiple polymer chains, multiple terminating R₃ groups and multiple terminating R₁ groups;
wherein R₁ is a terminating group chosen from ORₓ or NH-L-R⁺, wherein Rₓ is selected from the group consisting of H, alkyl, aryl, substituted alkyl, substituted aryl, carboxylic acid, ester, amide, amine, urethane, ether, and carbonyl and provided that at least some of the terminating R₁ groups are NH-L-R⁺;
wherein R₂ is a polymer chain; H; -C(=O)-CRₚ=CR_{q}Rᵣ, wherein Rₚ, R_{q}, Rᵣ are independently from one another, selected from the group consisting of H, alkyl, aryl, substituted alkyl, substituted aryl, carboxylic acid, ester, amide, amine, urethane, ether, and carbonyl; or -C(=O)NR_{d}-(CRₑR_{f})ₘ-O-C(=O)-CRₚ=CR_{q}Rᵣ, wherein Rₚ, R_{q}, Rᵣ, R_{d}, Rₑ and R_{f} are independently from one another, selected from the group consisting of H, alkyl, such as methyl or ethyl, aryl, such as phenyl, substituted alkyl, substituted aryl, carboxylic acid, ester , amide, amine, urethane, ether, and carbonyl and m is an integer greater than 1; provided that at least some of the R₂ groups are -C(=O)-CRₚ=CR_{q}Rᵣ or -C(=O)NR_{d}-(CRₑR_{f})ₘ-O-C(=O)-CRₚ=CR_{q}Rᵣ;
wherein R₃ is a terminating group chosen from H or -C(=O)-CRₚ=CR_{q}Rᵣ, wherein Rₚ, R_{q}, Rᵣ are independently from one another, selected from the group consisting of H, alkyl, aryl, substituted alkyl, substituted aryl, carboxylic acid, ester, amide, amine, urethane, ether, and carbonyl; or -C(=O)NR_{d}-(CRₑR_{f})ₘ-O-C(=O)-CRₚ=CR_{q}Rᵣ, wherein Rₚ, R_{q}, Rᵣ, R_{d}, Rₑ and R_{f} are independently from one another, selected from the group consisting of H, alkyl, such as methyl or ethyl, aryl, such as phenyl, substituted alkyl, substituted aryl, carboxylic acid, ester , amide, amine, urethane, ether, and carbonyl and m is an integer greater than 1; provided that at least some of the R₃ groups are -C(=O)-CRₚ=CR_{q}Rᵣ or -C(=O)NR_{d}-(CRₑR_{f})ₘ-O-C(=O)-CRₚ=CR_{q}Rᵣ;
wherein z is an integer from 1 to 20;
wherein n is an integer greater than 1.
When R₂ is "a polymer chain" this means that the polymer backbone continues from that point of attachment.

The pre-polymer is such that at least some of the terminating R₁ groups are NH-L-R⁺, at least some of the R₂ groups are -C(=O)-CRₚ=CR_{q}Rᵣ or -C(=O)NR_{d}-(CRₑR_{f})ₘ-O-C(=O)-CRₚ=CR_{q}Rᵣ, and at least some of the R₃ groups are -C(=O)-CR_{P}=CR_{q}Rᵣ or -C(=O)NR_{d}-(CRₑR_{f})ₘ-O-C(=O)-CRₚ=CR_{q}Rᵣ. The phrase "at least some" is used because there can be variability along the polymer chains of the pre-polymer and there can be variability between polymer chains of the pre-polymer. At least some of the terminating R₃ groups are NH-L-R⁺, so within a sample of the pre-polymer there will be polymer chains having R₁ groups that are NH-L-R⁺. Other R₁ groups (on other polymer chains or within the same polymer chain) are ORₓ groups. At least some of the R₂ groups are -C(=O)-CRₚ=CR_{q}Rᵣ or -C(=O)NRₐ-(CRₑR_{f})ₘ-O-C(=O)-CRₚ=CR_{q}Rᵣ, so within a sample of the pre-polymer there will be polymer chains having R₂ groups that are -C(=O)-CRₚ=CR_{q}Rᵣ or -C(=O)NR_{d}-(CRₑR_{f})ₘ-O-C(=O)-CRₚ=CR_{q}Rᵣ. Other R₂ groups are polymer chains or H. At least some of the R₃ groups are -C(=O)-CRₚ=CR_{q}Rᵣ or -C(=O)NR_{d}-(CRₑR_{f})ₘ-O-C(=O)-CRₚ=CR_{q}Rᵣ, so within a sample of the pre-polymer there will be polymer chains having R₃ groups that are -C(=O)-CRₚ=CR_{q}Rᵣ or -C(=O)NR_{d}-(CRₑR_{f})ₘ-O-C(=O)-CRₚ=CR_{q}Rᵣ. Other R₃ groups are H.

The R₁ groups are suitably chosen from the preferred groups of formula (I) shown above.

z is preferably from 2-10, more preferably from 4-10 and most preferably z is 8.

The value of n is suitably sufficiently large that the pre-polymer has a weight average molecular weight as described above, e.g. from about 1,000 Daltons to about 1,000,000 Daltons.

### Composition

The composition according to the present invention can be manufactured in the presence and/or mixed with a coloring agent. Preferred examples of coloring agents are the ones recommended by the FDA for use in medical devices, pharmaceutical products or cosmetics.

Similarly, the composition can further comprise stabilizers, for example 4-methoxyphenol (MEHQ), N-Phenyl-2-naphthylamine (PBN), phenothiazine (PTZ) and/or 5,5-Dimethyl-1-pyrroline N-oxide (DMPO).

The pre-polymer of the composition can be further reacted with one or more additional materials to modify the crosslinks between the polymer chains. For example, prior to or during curing/crosslinking, one or more hydrogel or other oligomeric or monomeric or polymeric precursors (e.g., precursors that may be modified to contain acrylate groups) such as poly(ethylene glycol), dextran, chitosan, hyaluronic acid, alginate, other acrylate based precursors including, for example, acrylic acid, butyl acrylate, 2-ethylhexyl acrylate, methyl acrylate, ethyl acrylate, acrylonitrile, n-butanol, methyl methacrylate, acrylic anhydride, methacrylic anhydride and TMPTA, trimethylol propane trimethacrylate, pentaerythritol trimethacrylate, pentaerythritol tetramethacrylate, ethylene glycol dimethacrylate, dipentaerythritol penta acrylate, Bis-GMA (bisphenol A-glycidyl methacrylate) and TEGDMA (tri-ethylene, glycol dimethacrylate), sucrose acrylate; other thiol based precursors (monomeric or polymeric); other epoxy based precursors; and combinations thereof, can be reacted with the acrylated pre-polymer.

The composition according to the present invention can be a surgical composition and is suitably used as a tissue sealant. The composition suitably has flow characteristics such that it can be applied to the desired area through a syringe or catheter but is sufficiently viscous to remain in place at the site of application without being washed away by bodily fluids, such as water and/or blood.

Preferably, the viscosity of the composition is 500 to 100,000 cP, more preferably 1,000 to 50,000 cP, even more preferably 2,000 to 40,000 cP. Viscosity analysis is performed using a Brookfield DV-II + Pro viscosimeter with a 2.2mL chamber and SC4-14 spindle, the speed during the analysis is varied from 5 to 80 rpm. The above-mentioned viscosity is present in the relevant temperature range for medical application i.e. room temperature and up to 45°C, preferably 37°C.

The composition of the invention may be incubated in bodily fluids, such as blood, prior to administration and curing, without a substantial decrease in fixing strength when cured.

The composition of the invention is suitably stable in bodily fluids, such as blood. More particularly, the composition of the invention suitably does not spontaneously crosslink in bodily fluids absent the presence of an intentionally applied stimulus such as light, for example UV light, heat, or chemical initiator to initiate crosslinking.

The composition of the invention is suitably stable when stored prior to use. Suitably the composition is stable for at least 3 months, preferably for at least 6 months and more preferably for at least 12 months. Stability in this context means that the pre-polymer does not degrade and the fixation performance is maintained. The inventors have observed that for some pre-polymers prepared according to prior art methods, the amount of positively charged heteroatom, particularly ammonium, degrades over time. This can be measured using ¹H NMR; the peak attributable to the ammonium decreases and a peak attributable to a free amine appears. Loss of positively charged heteroatom (e.g. ammonium groups) has been correlated with loss of performance (particularly loss of fixation strength). By contrast, the inventors have observed that with the pre-polymers according to the present invention there is no degradation of positively charged heteroatom, particularly ammonium groups, over time.

The composition can be cured using a free radical initiated reaction, such as, for example, by photo-initiated polymerization, thermally-initiated polymerization, and redox initiated polymerization.

Preferably, the composition of the invention is irradiated with light, for example ultraviolet (UV) and preferably with visible light (typically blue light or green light). Preferably the composition of the invention is irradiated with light in the presence of a photoinitiator to facilitate the reaction. Accordingly, in preferred embodiment, the composition of the invention contains a photoinitiator to facilitate the reaction. Examples of suitable photoinitiators include, but are not limited to: 2-dimethoxy-2-phenyl-acetophenone, 2-hydroxy-1-[4-(hydroxyethoxy)phenyl]-2-methyl-1-propanone (Irgacure 2959), 1-hydroxycyclohexyl-1-phenyl ketone (Irgacure 184), 2-hydroxy-2-methyl-1-phenyl-1-propanone (Darocur 1173), 2-benzyl-2-(dimehylamino)-1-[4-morpholinyl) phenyl]-1-butanone (Irgacure 369), methylbenzoylformate (Darocur MBF), oxyphenyl-acetic acid-2-[2-oxo-2-phenyl-acetoxy-ethoxy]-ethyl ester (Irgacure 754), 2-methyl-1-[4-(methylthio)phenyl]-2-(4-morpholinyl)-1-propanone (Irgacure 907), diphenyl(2,4,6-trimethylbenzoyl)-phosphine oxide ((e.g. Darocur TPO, Omnirad TPO), Ethyl(2,4,6-Trimethylbenzoyl)-phenyl phosphinate (Speedcure TPO-L, Omnirad TPO-L), phosphine oxide, phenyl bis(2,4,6-trimethyl benzoyl) (Irgacure 819), eosin Y disodium salt, N-Vinyl-2-Pyrrolidone (NVP) and triethanolamine, and camphorquinone, and combinations thereof.

In applications of the composition involving *in vivo* photopolymerization and other medical applications, the use of cytocompatible photoinitiators is preferred and may be required by regulatory agencies. Photoinitiator Omnirad TPO may be used, which causes minimal cytotoxicity (cell death) over a broad range of mammalian cell types and species.

In order for the photopolymerization to occur, the composition (and the substrate to which the composition is applied, if applicable) is preferably sufficiently transparent to the light.

In applications when the composition is cured *in vivo,* the temperature at which curing occurs is preferably controlled as not damage the tissue on which the composition has been applied. Preferably, the composition is not heated above 45°C during irradiation, more preferably not above 37°C.

In addition to photochemical crosslinking, the composition can be cured thermally, by Mitsunobu-type reaction, by redox-pair initiated polymerization for example benzoyl peroxide, N,N,-dimethyl-p-toluidine, ammonium persulfate, or tetramethylenediamine (TEMED), and by a Michael-type addition reaction using a bifunctional sulfhydryl compound.

In one embodiment, a composition according to the invention may comprise the redox initiator system comprising:
- at least one oxidant selected from the group consisting of APS (Ammonium persulfate), KPS (Potassium persulfate) and BPO (Benzoyl peroxide);
- at least one reducing agent selected from the group consisting of TMA (4-N,N Trimethylaniline), N,N-Bis(2-hydroxyethyl)-p-toluidine, N,N-Dimethylaniline, N,N-Diethylaniline, sodium p-toluenesulfonate, N-Methyl-N-(2-hydroxyethyl)-p-toluidine, MHPT (N-(2-Hydroxyethyl)-N-methyl-para-toluidine) and Phosphine (Diphenylphosphinostryrene or triphenylphosphine);
- and at least one radical scavenger selected from the group consisting of Tempol and or 4-methoxyphenol.

According to a special embodiment, a redox composition may comprise 0.1 to 5 wt% of a reducing agent, e.g., 4-N,N Trimethylaniline, N,N-Bis(2-hydroxyethyl)-p-toluidine, N,N-Dimethylaniline, N,N-Diethylaniline, sodium p-toluenesulfonate or N-Methyl-N-(2-hydroxyethyl)-p-toluidine; 0 to 5 wt% of an oxygen inhibitor, e.g., 4-(Diphenylphosphino)styrene or triphenylphosphine; 0.005 to 0.5 wt% of a working time agent, e.g., Tempol or 4-methoxyphenol; and 0.1 to 10 wt% of an oxidant, e.g., ammonium persulfate, potassium persulfate or benzoyl peroxide.

Upon polymerization, the pre-polymer forms a crosslinked network with improved properties and exhibits significant fixation strength even in the presence of blood and other bodily fluids. The cured polymer is preferably sufficiently elastic to resist movement of the underlying tissue, for example contractions of the heart and blood vessels. The cured polymer can provide a seal, preventing the leakage of fluids or gas. The cured polymer is preferably biodegradable and biocompatible, causing minimal inflammatory response. The cured polymer is preferably elastomeric.

Biodegradability can be evaluated *in vitro,* such as in phosphate buffered saline (PBS) or in acidic or alkaline conditions. Biodegradability can also be evaluated *in vivo,* such as in an animal, for example mice, rats, dogs, pigs or humans. The rate of degradation can be evaluated by measuring the loss of mass of the polymer over time *in vitro* or *in vivo.*

The cured composition, alone or coated on a patch or tissue suitably exhibits a 90° pull off fixation strength of at least 0.5 N/cm², preferably at least 1 N/cm² and even more preferably at least 2 N/cm², for example 1.5 N/cm² to 2 N/cm², but preferably greater than 5 N/cm², for example up to 6 N/cm² or 7 N/cm² or greater. Pull off fixation strength refers to the fixation value obtained by attaching an article or sample to wet tissue, such as epicardial surface of cardiac tissue or blood vessels immobilized on a flat substrate, such as a metallic stub. The 90° pull off fixation test determines the greatest perpendicular force (in tension) that a surface area can bear before detachment (N. Lang et al., Sci. Transl. Med., 2014, 6, 218ra6).

According to preferred embodiment, the composition of the invention is cured in light and in presence of a photo initiator and the cured composition exhibits a 90° pull off fixation strength of at least 0.5 N/cm², preferably at least 1 N/cm² and even more preferably at least 2 N/cm², for example 1.5 N/cm² to 2 N/cm², but preferably greater than 5 N/cm², for example up to 6 N/cm² or 7 N/cm² or greater.

The cured composition can desirably also exhibit a burst pressure of greater than 100 mmHg, preferably in the range of 400 mmHg to 600 mmHg or greater, for example 400mmHg or 500mmHg. Burst pressure or strength refers to the pressure value obtained to burst an explanted porcine carotid arterial vessel, which has an incision coated with the composition.

The composition of the present invention when cured in light and in the presence of a photo-initiator preferably has one or more of the following properties:
i) 90° pull off strength greater than 0.5 N/cm², preferably 2 to 7 N/cm² or greater; and
ii) burst performance of greater than 100 mmHg, preferably 200 to 300 mmHg or greater.

According to preferred embodiment, the composition of the invention is able after curing to fix strongly to a surface or to fix one surface to another.

According to an alternative embodiment, the composition of the invention is used as sealant, i.e., it is able after curing to prevent leaking (e.g., of fluid or gas) by forming a barrier or filling a void volume.

Besides fixation and sealing of wet biological tissue, the composition may fix to and seal a variety of hydrophilic or hydrophobic substrates, natural or synthetic, including polyethylene terephthalate, expanded polyethylene terephthalate, polyester, polypropylene, silicones, polyurethanes, acrylics, fixed tissue (e.g., pericardium), ceramics or any combinations thereof.

### Method of preparation

The method for preparing the composition of the present invention, comprises several required steps, which may accommodate several variations. According to a preferred embodiment, said method comprises the steps of:
i) providing a pre-polymer having a polymeric backbone comprising a polymeric unit of the formula (-A-B-)ₙ, wherein A is derived from a substituted or unsubstituted polyol or mixture thereof and B is derived from a substituted or unsubstituted polyacid or mixture thereof, and n is an integer greater than 1;
ii) reacting the pre-polymer with an amine of formula NH₂-L-R to provide a pre-polymer comprising groups of formula (II): 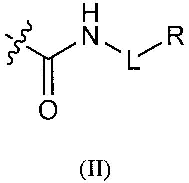 wherein L is a linking moiety, and wherein R is a moiety comprising a heteroatom;
iii) activating the pre-polymer comprising the groups of formula (II) to provide an activated pre-polymer; and
iv) reacting the activated pre-polymer with an acid to provide a pre-polymer comprising groups of formula (I): 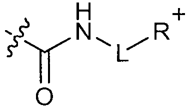 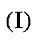 wherein R⁺ is a moiety comprising a positively charged heteroatom.

Step (i) of providing a pre-polymer may be a polymerization step wherein monomers are reacted to provide the pre-polymer backbone. Alternatively, step (i) may be the provision of a commercially available pre-polymer such as polyglycerol sebacate.

In a polymerization step the monomers comprise component A (polyol or a mixture of polyols) and component B (polyacid or a mixture of polyacids) and are suitably added together in a molar ratio range of 0.5:1 to 1.5:1, preferably 0.9:1.1 and most preferably 1:1. Where component A is glycerol and component B is sebacic acid and added in a 1:1 molar ratio, there are three hydroxy groups on glycerol for two carboxyl groups on the sebacic acid. Therefore, an extra hydroxy group on glycerol is available for activation, as well as terminal carboxylic acid groups.

The conditions for polymerization may include a temperature range of 100 to 140°C, preferably 120 to 130°C, an inert atmosphere, preferably comprising nitrogen, and under vacuum.

The pre-polymer resulting from step (i) will comprise terminal carboxylic groups.

In step (ii) the pre-polymer is reacted with an amine. The amine is of formula NH₂-L-R. The heteroatom in the R moiety may be derived from any element other than carbon or hydrogen. Preferred heteroatoms are nitrogen, phosphorus and sulfur. Most preferably, the heteroatom is a nitrogen atom.

In one embodiment the R moiety is an amine, preferably an amine of formula -NR^{a}R^{b} wherein the nitrogen forms a bond with L, and R^{a} and R^{b} are independently chosen from C₁₋₂₀ alkyl, benzyl, substituted benzyl, cycloalkyl or substituted cycloalkyl, or R^{a} and R^{b} are linked such that R^{a} and R^{b} form a saturated cyclic group having 2 to 20 carbon atoms. L is a linking moiety suitably chosen from a C₁₋₂₀ alkyl group, a C₂₋₂₀ alkenyl group, a C₂₋₂₀ alkynyl group, or a C₇₋₂₀ benzyl group wherein the groups are optionally substituted, and wherein the groups may optionally contain heteroatoms such as oxygen, nitrogen or sulfur. L is preferably a C₁₋₆ alkyl group. Alternatively, L may be an ethylene glycol chain, wherein the ethylene glycol chain is optionally substituted.

Preferred amines of formula (II) are shown below:

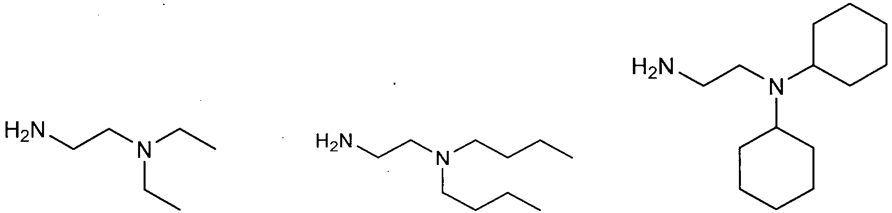

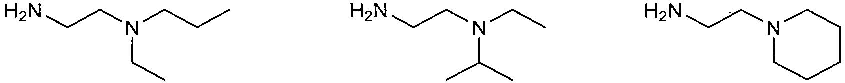

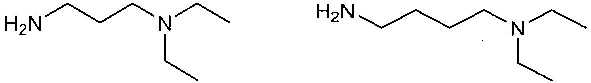

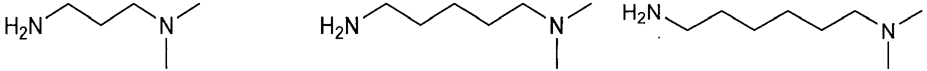

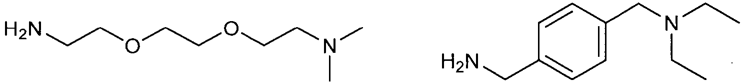

The amination may be carried out using standard techniques. Suitable solvents include dichloromethane (DCM), tetrahydrofuran (THF), N,N'-dimethylformamide (DMF), 2-methyltetrahydrofuran (MeTHF), ethyl acetate (EtOAc), N-methyl-2-pyrrolidone (NMP), toluene and acetonitrile. Suitable coupling agents include thionyl chloride (SOCl2), oxalyl chloride ((COCl)₂), phosphorus oxychloride, 1,1'-carbonyldiimidazole (CDI), 1-ethyl-3-(3-dimethylaminopropyl)carbodiimide (EDCI), dicyclohexylcarbodiimide (DCC), N,N'-diisopropylcarbodiimide (DIC) propylphosphonic anhydride (T3P), benzotriazole-1-yloxytripyrrolidinophosphonium hexafluorophosphate (PyBOP), N-[(Dimethylamino)-1H-1,2,3-triazolo[4,5-b]-pyridin-1-ylmethylene]-N-methylmethanaminium Hexa-fluorophosphate N-Oxide (HATU), HBTU, and TBTU. Suitable catalysts include 4-Dimethylaminopyridine (DMAP), 1-hydroxybenzotriazole (HOBt), 3-hydroxy-4-oxo-3,4-dihydro-1,2,3-benzotriazine (HOOBt), N,N'-dimethylformamide (DMF), 2-hydroxypyridine-N-oxide (HOPO), and pyridine.

In one embodiment the amount of amination may be controlled by varying the amount of coupling agent.

The activation in step iii) is suitably achieved by acrylation of the pre-polymer backbone.

In a preferred embodiment, the activation is done through acrylation of hydroxy groups on the pre-polymer backbone.

One or more acrylates may be used as the acrylating agent. The acrylate may contain the following group: -C(=O)-CRₚ=CR_{q}Rᵣ, wherein Rₚ, R_{q}, Rᵣ are independently from one another, selected from the group consisting of H, alkyl such as methyl or ethyl, aryl such as phenyl, substituted alkyl, substituted aryl, carboxylic acid, ester, amide, amine, urethane, ether, and carbonyl. Preferably Rₚ is H. Alternatively the acrylate may contain the following group: According to another embodiment, acrylate groups may contain the following group: - C(=O)NR_{d}-(CRₑR_{f})ₙ-O-C(=O)-CRₚ=CR_{q}Rᵣ, wherein Rₚ, R_{q}, Rᵣ, R_{d}, Rₑ and R_{f} are independently from one another, selected from the group consisting of H, alkyl, such as methyl or ethyl, aryl, such as phenyl, substituted alkyl, substituted aryl, carboxylic acid, ester, amide, amine, urethane, ether, and carbonyl; and n is an integer greater than 1 (e.g. n is 2).

The acrylating agent may be acryloyl chloride.

Alternatively, activation in step iii) can be acrylation using an isocyanate acrylate compound. A preferred isocyanate acrylate compound is 2-isocyanatoethyl(meth)acrylate.

Acrylation may be carried out using standard techniques. Suitable solvents include dichloromethane (DCM). Suitable coupling agents include triethylamine (TEA). Suitable catalysts include 4-Dimethylaminopyridine (DMAP).

In one embodiment the amount of amination may be controlled by varying the amount of acryloyl chloride.

In step (iv) the activated pre-polymer is reacted with an acid. The acid may be a carboxylic acid, such as formic acid or acetic acid, or may be hydrochloric acid.

At least one additive may be added to the composition obtained at step (iv). In a preferred embodiment, said additive is selected from the group consisting of photoinitiators, radical inhibitors, and dyes.

According to a preferred embodiment, the method further comprises one or more purification steps (v) to ensure that solvents, by-products, impurities, or un-reacted products are removed from the composition. These may be conducted throughout any reaction steps and more than one purification technique may be applied during the preparation of the composition.

In a preferred embodiment, such purification steps may include washes in aqueous media. Phase separation during water washings can be improved by the use of salts solubilized in the aqueous phase (e.g. from about 50 to about 500 g/L salt aqueous solution, preferably about 300 g/L salt, for example sodium chloride, aqueous solution). According to a preferred embodiment, the water washing is salted water washing. Examples of salts include, but are not limited to, sodium chloride or potassium chloride.

Alternatively, purification steps may be carried out using acidic or basic aqueous solutions such as aqueous hydrogen chloride solution (from about 0.1N to 5N) or aqueous sodium carbonate solution (from about 10% w/w to saturated).

According to a preferred embodiment, such purification steps may be conducted either by solvent evaporation or supercritical carbon dioxide extraction.

When the R⁺ moiety is of formula -N⁺R^{a}R^{b}R^{c} a method according to the invention may comprise the steps of:
i) providing a pre-polymer having a polymeric backbone comprising a polymeric unit of the formula (-A-B-)ₙ, wherein A is derived from a substituted or unsubstituted polyol or mixture thereof and B is derived from a substituted or unsubstituted polyacid or mixture thereof, and n is an integer greater than 1;
ii) reacting the pre-polymer with a compound of formula NH₂-L-N⁺R^{a}R^{b}R^{c}; and
iii) activating the pre-polymer to provide an activated pre-polymer.

The compound of formula NH₂-L-N⁺R^{a}R^{b}R^{c} could be, e.g. (2-aminoethyl)trimethylammonium chloride hydrochloride.

Alternatively, when the R⁺ moiety is of formula -N⁺R^{a}R^{b}R^{c} a method according to the invention may comprise the steps of:
i) providing a pre-polymer having a polymeric backbone comprising a polymeric unit of the formula (-A-B-)ₙ, wherein A is derived from a substituted or unsubstituted polyol or mixture thereof and B is derived from a substituted or unsubstituted polyacid or mixture thereof, and n is an integer greater than 1;
ii) reacting the pre-polymer with an amine of formula NH₂-L-NR^{a}R^{b};
iii) alkylation with an alkylating agent; and
iv) activating the pre-polymer to provide an activated pre-polymer.

Such a method may incorporate the Menshutkin reaction and may use an alkylating agent such as methyl iodide.

### Uses

### Tissue fixation and sealing

The composition according to the invention may be used for joining or sealing targeted surfaces including tissue, graft material such as PTFE-based graft, medical devices, surgical patch or any combination thereof. The method for joining or sealing targeted surfaces comprises applying the composition to the surface and curing the composition.

Unlike conventional tissue adhesives that spontaneously activate during application or in the presence of water, or adhesives that are hydrophilic and thus are subject to washout prior to curing, the composition according to the invention can be applied to wet substrates without activation or displacement. The composition can also be applied to dry substrates.

The composition may also be used for joining tissue to the surface of a medical device or maintaining medical device, for example a biocompatible implant (e.g. nerve conduit) in place *in vivo.* The composition can be used in medical devices, either as part or all of a device or to fix a device to tissue. The method for joining tissue to the surface of a medical device comprises applying the composition to the surface of the tissue and/or medical device and curing the composition. The composition can also be used to join tissue, including one or more tissue *in vivo.*

Surgical compositions comprising the composition according to the invention can also be used for other applications. Examples of applications include to stop bleeding, for example, due to a wound or trauma, during surgery such as after suturing a graft to a vessel, or after vascular access in endovascular procedures. The composition does not need to be removed before the surgeon sutures the wound closed since it will degrade over time. Other types of wounds that can be treated include, but are not limited to, wounds that leak, wounds that are hard to close or that fail to heal properly through normal physiologic mechanisms. The application can be performed both inside or outside the body, for human or veterinary use.

The composition according to the invention can also be fabricated into a biodegradable stent. The stent can increase the diameter of a blood vessel to increase flow through the vessel, but since the stent is biodegradable, the blood vessel can increase in diameter with a reduced risk of thrombosis or covering the stent with scar tissue, which can re-narrow the blood vessel. The composition can cover an outer surface of a stent to help fix the stent to a vessel wall in a manner that is less damaging to the tissue than an uncovered stent or avoid its displacement inside the body. Similarly, the composition can cover the surface of any devices which are in contact with tissue to provide a suitable interface that can join to tissue.

The composition according to the present invention can be used in a variety of other applications where fixing or sealing is required. These include, but are not limited to, air leaks following a lung resection; to reduce the time for surgical procedures; to seal dura; to ease laparoscopic procedures; as a degradable skin composition; as a hernia matrix to prevent or to reduce the need for stables or tacks; to prevent blood loss; to manipulate organs or tissues during surgical procedures; to secure corneal transplants in place; to patch a heart to deliver drugs and/or to reduce dilation of the heart after myocardial infarction; to attach another material to a tissue; to augment sutures or staples; to distribute forces across tissue; to prevent leaks; as a barrier membrane on the skin to prevent evaporation of water from burnt skin; as a patch for delivery of anti-scar or antimicrobial medication; to attach devices to tissue; to attach devices to mucus membrane as a tape to secure devices within an oral cavity, such as to hold dentures and oral appliances; as a tape to anchor soft tissue to bone; to prevent the formation of holes in tissue; and to enhance/augment mechanical properties of tissues, etc.

### Delivery of bioactive molecules

The composition according to the invention described may also contain one or more pharmaceutical, therapeutic, prophylactic, and/or diagnostic agents that are released during the time period that the material functions as a sealant or fixing composition. The agent may be a small molecule agent, for example, having molecular weight less than 2000, 1500, 1000, 750, or 500 Daltons, a biomolecule, for example peptide, protein, enzyme, nucleic acid, polysaccharide, growth factors, cell adhesion sequences, such as RGD sequences or integrins, extracellular matrix components, or combinations thereof. Exemplary classes of small molecule agents include, but are not limited to, anti-inflammatories, analgesics, antimicrobial agents, and combinations thereof. Exemplary growth factors include, without limitation, TGF-β, acidic fibroblast growth factor, basic fibroblast growth factor, epidermal growth factor, IGF-I and II, vascular endothelial-derived growth factor, bone morphogenetic proteins, platelet-derived growth factor, heparin-binding growth factor, hematopoetic growth factor, peptide growth factor, or nucleic acids. Exemplary extracellular matrix components include, but are not limited to, collagen, fibronectin, laminin, elastin and combinations thereof. Proteoglycans and glycosaminoglycans can also be covalently or non-covalently associated with the composition of the present invention.

### Tissue support

The composition according to the invention can be used to create tissue repair supports to serve a mechanical function within the body of a patient in need thereof.

The present invention further provides methods of use and use of the composition according to the present invention for preparing printing resin (e.g. 3D printing resin) and for producing shaped objects by a variety of techniques known in the art, including 3D printing. The invention further provides printed (e.g. 3D printed) shaped objects obtained by using the composition of the invention. Accordingly, the composition of the invention can be used for forming shaped articles such as for example biocompatible implant (e.g. nerve conduit). The shaped articles may be produced by a variety of fabrication techniques known in the art, including 3D printing. Such articles may exert functions, such as holding two tissues together or positioning the tissue in a specific position inside or outside the body. The shaped object may have micro or nanoscale resolution.

According to another embodiment, the composition of the invention is applied to any substrate having a surface, more particularly a tissue repair support, such as a surgical patch, for example a mesh substrate (e.g. hernia mesh substrate).

According to another embodiment, tissue can be coated with a layer of the composition of the invention, for example, the lumen of a tissue, such as a blood vessel to prevent restenosis, reclosure or vasospasm after vascular intervention.

The composition may also contain one or more types of cells, such as connective tissue cells, organ cells, muscle cells, nerve cells, and combinations thereof. Optionally, the material is seeded with one or more of tenocytes, fibroblasts, ligament cells, endothelial cells, lung cells, epithelial cells, smooth muscle cells, cardiac muscle cells, skeletal muscle cells, islet cells, nerve cells, hepatocytes, kidney cells, bladder cells, urothelial cells, chondrocytes, and bone-forming cells. The combination of cells with the material may be used to support tissue repair and regeneration.

### Anti-adhesion barriers

The composition according to the invention herein described can be applied to reduce or prevent the formation of adhesions after surgical procedures. For example, the composition can be applied to prevent adhesion of brain tissue to the skull after brain surgery or implantation of devices to prevent peritoneal adhesion.

### Other applications

The compositions can also be used to coat tools, such as surgical instruments, for example, forceps or retractors, to enhance the ability of the tools to manipulate objects. The compositions can also be used in industrial applications where it is useful to have a degradable material that is biocompatible, for example, to reduce potential toxicity of the degradation products, such as marine applications, for example, in underwater use or attaching to the surface of boats.

The present invention will now be illustrated, but in no way limited, by reference to the following examples.

### EXAMPLES

### Comparative Example 1: Acrylated and aminated poly(glycerol sebacate)

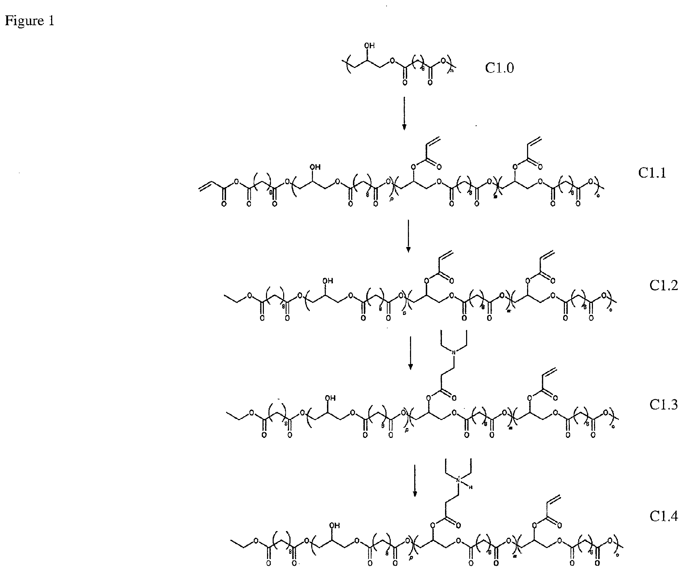

Acrylated and aminated poly(glycerol sebacate) was made according to the methods disclosed in WO 2021/078962. The synthetic route is shown in Figure 1. Poly(glycerol sebacate) (C1.0 in Figure 1) was reacted with acryloyl chloride and then with ethanol to provide poly(glycerol sebacate) acrylate (C1.2 in Figure 1). Further reaction with diethylamine and then with acetic acid provided aminated and acrylated poly(glycerol sebacate) (C1.4 in Figure 1).

### Example 1: Poly(glycerol sebacate) with amide bonds

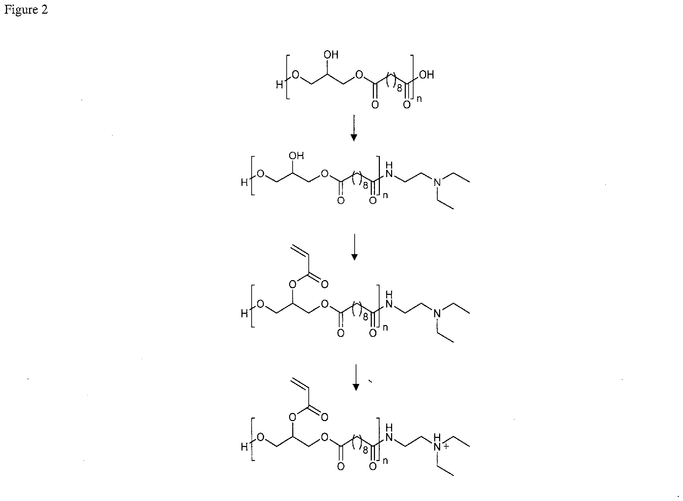

The synthetic steps of Example 1 are illustrated in Figure 2.

### (i) Synthesis of poly(glycerol sebacate) (PGS):

1. Equimolar amounts of glycerol and sebacic acid were weighed.
2. The reaction mixture temperature set between 120 and 130°C until the monomers were completely melted.
3. Upon melting of the reagents, the bath or reaction temperature was reduced to the target value of 120°C and stirring started.
4. The air inside the flask was replaced with nitrogen using three vacuum/purging cycles.
5. The reaction was followed for 8 hours.
6. The nitrogen supply was then removed, and the pressure reduced using a vacuum pump set to a target of 15 mBars.

The reaction was followed until the targeted Mw (about 5,000 Da) and polydispersity (< 3) were achieved. The glycerol : sebacic acid molar ratio targeted was 1:1, as confirmed by proton nuclear magnetic resonance (¹H-NMR).

### (ii) Formation of amide bonds (Synthesis of PGS.DEDA)

1. Dichloromethane (DCM) was added to a mixture of PGS, N,N-Diethylethylenediamine (DEDA, 2 mol. eq./COOH), and 4-Dimethylaminopyridine (DMAP, 0.5 mol. eq./COOH) and stirred at room temperature until complete dissolution.
2. Once a transparent solution was obtained, 1-ethyl-3-(3-dimethylaminopropyl)carbodiimide (EDCI) was added to the reaction mixture and the system was stirred for 24h at room temperature.
3. The reaction mixture was diluted to a concentration of 7wt.% of polymer by adding the corresponding volume of DCM.
4. The solution was washed three times with 0.3 volume equivalents of HCl (0.5N) solution, once with 0.3 volume equivalents of saturated NaHCO₃ solution and once with 0.3 volume equivalents of distilled water.
5. After the final wash, the system was allowed to separate. The two organic layers that formed were collected together, and the solvent was evaporated under reduced pressure.
6. The recovered transparent viscous liquid was dried under vacuum in presence of phosphorous pentoxide (yield: 60-70%).

The obtained functionalized polymer was analyzed using size exclusion chromatography (SEC) with tetrahydrofuran (THF) and proton nuclear magnetic resonance (¹H NMR) in deuterated dichloromethane (CD₂Cl₂) for molecular weight and structural characterization, respectively.

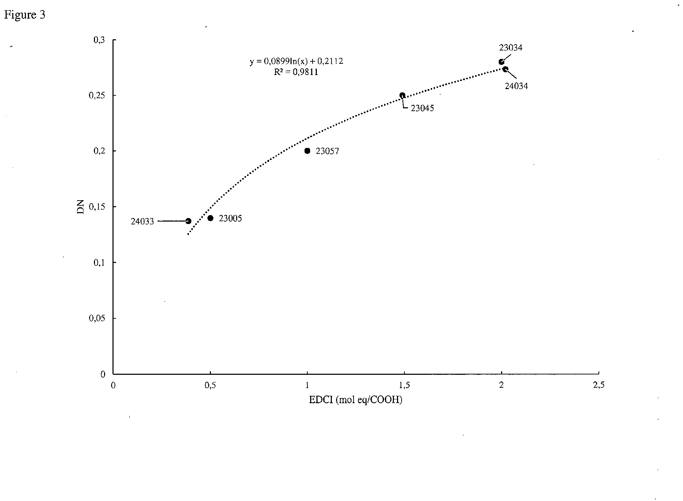

By varying the quantity of EDCI, with all other parameters being fixed, it was possible to adjust the amount of amide bonds formed. Figure 3 shows the calibration curve showing how the amount of amide bonds (shown as DN) varied with the quantity of EDCI used in the reaction. The curve was fitted with a logarithmic function.

### (iii) Acrylation (Synthesis of PGSA.DEDA)

1. A solution mixture of PGS.DEDA (3.6 wt.%), triethylamine (TEA, 1.1 mol. eq./acryloyl chloride) and DMAP (catalytic amount) was prepared in dry DCM.
2. The solution was cooled down between 0 and 5°C, put under nitrogen flow and stirred.
3. A solution of acryloyl chloride (1.3N) in dry DCM was added dropwise at a rate such that the solution was maintained between 0 and 5°C.
4. The reaction mixture was stirred at room temperature for 20h.
5. Ethanol (1.73 wt. eq./polymer) was added, and the reaction mixture was put under reflux at 40°C and stirred for 5h.
6. The reaction mixture was washed three times with 0.3 volume equivalents of NaCl (150g/L) solution.
7. The organic layer was recovered and directly used for acidification.

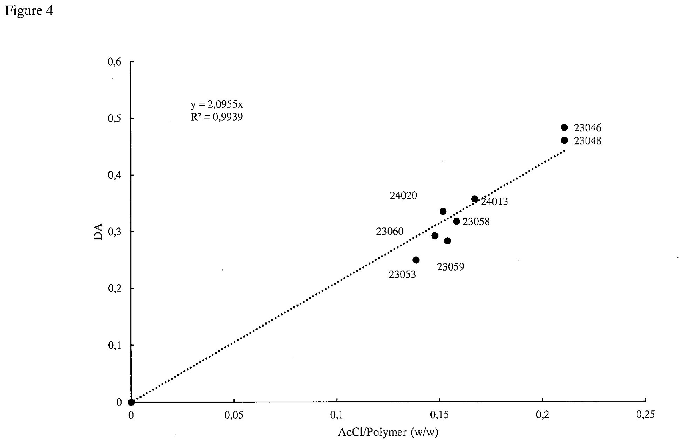

By varying the quantity of acryloyl chloride, with all other parameters being fixed, it was possible to adjust the amount of acrylate groups formed. Figure 4 shows how the amount of acrylate (shown as DA) varied with the quantity of acryloyl chloride used in the reaction. The curve was fitted with a straight line.

### (iv) Acidification

1. AcOH (1.5 mol.eq./DEDA) was added to the reaction mixture and stirred at room temperature for 15min.
2. The reaction mixture was washed three times with 0.3 volume equivalents of NaCl (150g/L) solution.
3. The organic layer was recovered, dried using MgSO₄, filtered through Whatman filter and the solvent was removed under reduced pressure until the pre-polymer concentration reached approximately 50 wt.%.

### (v) Formulation and Purification

1. To prepare the formulation, 8000 ppm of TPO and 1000 ppm of MEHQ were added to the pre-polymer solution. The mixture was then stirred for at least 15min at room temperature until complete dissolution.
2. The formulation was then purified using 100 mass equivalents of supercritical carbon dioxide (ScCO₂) compared to the mass of solvent. This purification took place in a ScCO₂ reactor at a temperature of 40°C and a pressure of 250 bar. The flow rate of CO₂ was maintained at 20 grams per minute.
3. The resulting purified pre-polymer was collected as a white foam in an amber vial, which was then placed under reduced pressure (15mbar) for about 1h to remove CO₂.

The pre-polymer formulation was analyzed using size exclusion chromatography (SEC) with tetrahydrofuran (THF) and proton nuclear magnetic resonance (¹H NMR) in deuterated dichloromethane (CD₂Cl₂) for molecular weight and structural characterization, respectively.

Table 1 below shows the characterization data for samples of acrylated and aminated poly(glycerol sebacate) (comparative example 1) and eight samples of poly(glycerol sebacate) with amide bonds (examples 1A-1H). The values for comparative example 1 are average values across a range of formulations.

**Table 1**

| | M_{w} (kg/mol) | Polydispersity | DA | DN | Viscosity (cP) |
|---|---|---|---|---|---|
| Comparative Example 1 | 2.7-5.3 | <2.5 | 0.27-0.37 | 0.14-0.24 | 15,000-35,000 |
| Example 1A | 2.04 | 2.19 | 0.48 | 0.27 | 31558 |
| Example 1B | 2.06 | 2.03 | 0.46 | 0.29 | 39720 |
| Example 1C | 2.69 | 2.24 | 0.25 | 0.18 | 25293 |
| Example 1D | 2.76 | 2.37 | 0.32 | 0.21 | 26313 |
| Example 1E | 4.22 | 2.78 | 0.28 | 0.15 | 23355 |
| Example 1F | 2.43 | 2.23 | 0.29 | 0.23 | 17718 |
| Example 1G | 2.44 | 2.37 | 0.34 | 0.21 | 28313 |
| Example 1H | 2.14 | 2.21 | 0.34 | 0.20 | 24285 |

### Accelerated stability assessment and fixation strength assessment

Seven batches were conditioned in a 100 mL amber vial, which was then flushed with nitrogen and sealed with Parafilm. Prior to launching the accelerated stability test, the fixation strength for each batch was determined using the pull off test method on pericardial porcine tissue. Table 2 shows the fixation strength for the comparative examples and examples:

**Table 2**

| | Fixation Strength (N/cm²) |
|---|---|
| Comparative Example 1 | ≥5 |
| Example 1A | 3.9 (1.8) |
| Example 1B | 1.5 (1.1) |
| Example 1C | 8.5 (2.0) |
| Example 1D | 7.2 (1.9) |
| Example 1E | 8.8 (1.7) |
| Example 1F | 7.4 (2.4) |
| Example 1G | 6.3 (1.0) |
| Example 1H | 5.1 (1.3) |

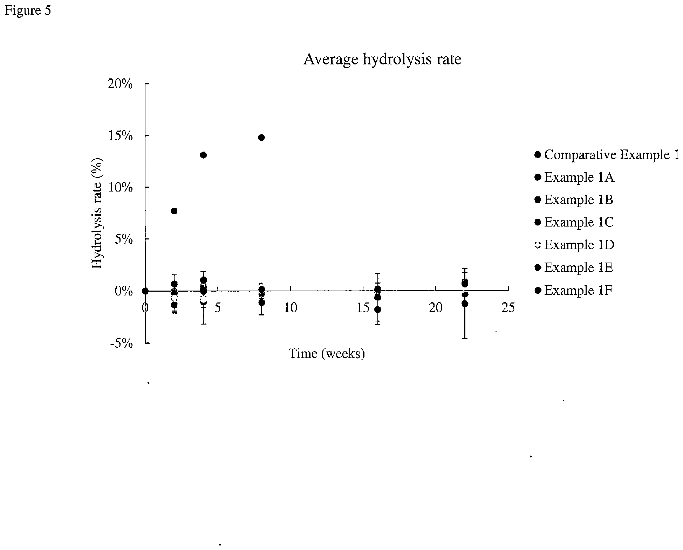

Subsequently, the prototypes were incubated in an oven at 45°C for 22 weeks. At predetermined time points, structural analysis was performed using 1D NMR (¹H, ¹³C), and 2D NMR (COSY, HSQC) on each batch. Specifically, stability of the amide function was monitored overtime. Figure 5 illustrates the evolution of the average hydrolysis rate evolution of amide functions, as determined by ¹H-NMR for six PGSA.DEDA batches. Additionally, it shows the average hydrolysis rate of the ester function for one acrylated and aminated poly(glycerol sebacate) (PGSAA) batch. It is evident that ester hydrolysis occurs in PGSAA, while amide hydrolysis is absent in PGSA.DEDA batches after 22 weeks of incubation at 45°C. This indicates the stability of the DN during the entire incubation period.

## Claims

1. A composition comprising:
a pre-polymer having a polymeric backbone comprising a polymeric unit of the formula (-A-B-)ₙ, wherein A is derived from a substituted or unsubstituted polyol or mixture thereof and B is derived from a substituted or unsubstituted polyacid or mixture thereof, and n is an integer greater than 1,
wherein the pre-polymer has activated groups on its polymeric backbone,
and wherein the pre-polymer further comprises groups of formula (I): 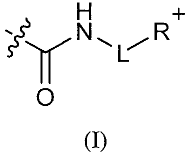
wherein L is a linking moiety, and wherein R⁺ is a moiety comprising a positively charged heteroatom.

2. The composition according to claim 1, wherein the positively charged heteroatom is a positively charged nitrogen, phosphorus or sulfur atom, preferably a positively charged nitrogen atom.

3. The composition according to claim 2, wherein the R⁺ moiety is of formula -N⁺R^{a}R^{b}₂H wherein R^{a} and R^{b} are independently chosen from C₁₋₂₀ alkyl groups, benzyl, substituted benzyl, cycloalkyl or substituted cycloalkyl, or R^{a} and R^{b} are linked such that R^{a} and R^{b} form a saturated cyclic group having 2 to 20 carbon atoms.

4. The composition according to claim 2, wherein the R⁺ moiety is of formula -N⁺R^{a}R^{b}R^{c} wherein R^{a}, R^{b} and R^{c} are independently chosen from C₁₋₂₀ alkyl groups, benzyl, substituted benzyl, cycloalkyl or substituted cycloalkyl, or at least two of R^{a}, R^{b} and R^{c} are linked such that R^{a}, R^{b} and/or R^{c} form a saturated cyclic group having 2 to 20 carbon atoms.

5. The composition according to any preceding claim, wherein L is chosen from a C₁₋₂₀ alkyl group, a C₂₋₂₀ alkenyl group, a C₂₋₂₀ alkynyl group, or a C₇₋₂₀ benzyl group wherein the groups are optionally substituted, and wherein the groups may optionally contain heteroatoms such as oxygen, nitrogen or sulfur, or L may be an ethylene glycol chain, wherein the ethylene glycol chain is optionally substituted.

6. The composition according to any preceding claim, wherein the proportion of groups of formula (I) compared to the number of A-B monomer units in the polymeric backbone is from about 0.05 to about 0.55 mol/mol of monomer unit.

7. The composition according to any preceding claim, wherein the polyol is a triol, preferably glycerol or trimethylolpropane ethoxylate, and wherein the polyacid is a diacid selected from the group consisting of glutaric acid, adipic acid, pimelic acid, sebacic acid and azelaic acid, preferably sebacic acid.

8. The composition according to any preceding claim, wherein the activated groups are acrylate groups or vinyl groups.

9. The composition according to any preceding claim, wherein the pre-polymer is of formula (A): 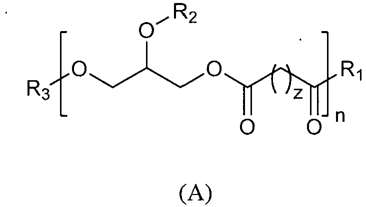
wherein the pre-polymer is branched such that there are multiple polymer chains, multiple terminating R₃ groups and multiple terminating R₁ groups;
wherein R₁ is a terminating group chosen from ORₓ or NH-L-R⁺, wherein Rₓ is selected from the group consisting of H, alkyl, aryl, substituted alkyl, substituted aryl, carboxylic acid, ester, amide, amine, urethane, ether, and carbonyl and provided that at least some of the terminating R₁ groups are NH-L-R⁺;
wherein R² is a polymer chain; H; -C(=O)-CRₚ=CR_{q}Rᵣ, wherein Rₚ, R_{q}, Rᵣ are independently from one another, selected from the group consisting of H, alkyl, aryl, substituted alkyl, substituted aryl, carboxylic acid, ester, amide, amine, urethane, ether, and carbonyl; or -C(=O)NR_{d}-(CRₑR_{f})ₘ-O-C(=O)-CRₚ=CR_{q}Rᵣ, wherein Rₚ, R_{q}, Rᵣ, R_{d}, Rₑ and R_{f} are independently from one another, selected from the group consisting of H, alkyl, such as methyl or ethyl, aryl, such as phenyl, substituted alkyl, substituted aryl, carboxylic acid, ester , amide, amine, urethane, ether, and carbonyl and m is an integer greater than 1; provided that at least some of R² groups are -C(=O)-CRₚ=CR_{q}Rᵣor -C(=O)NR_{d}-(CRₑR_{f})ₘ-O-C(=O)-CRₚ=CR_{q}Rᵣ;
wherein R³ is a terminating group chosen from H or -C(=O)-CRₚ=CR_{q}Rᵣ, wherein Rₚ, R_{q}, Rᵣ are independently from one another, selected from the group consisting of H, alkyl, aryl, substituted alkyl, substituted aryl, carboxylic acid, ester, amide, amine, urethane, ether, and carbonyl; or -C(=O)NR_{d}-(CRₑR_{f})ₘ-O-C(=O)-CRₚ=CR_{q}Rᵣ, wherein Rₚ, R_{q}, Rᵣ, R_{d}, Rₑ and R_{f} are independently from one another, selected from the group consisting of H, alkyl, such as methyl or ethyl, aryl, such as phenyl, substituted alkyl, substituted aryl, carboxylic acid, ester , amide, amine, urethane, ether, and carbonyl and m is an integer greater than 1; provided that at least some of R² groups are -C(=O)-CRₚ=CR_{q}Rᵣ or -C(=O)NR_{d}-(CRₑR_{f})ₘ-O-C(=O)-CRₚ=CR_{q}Rᵣ;
wherein z is an integer from 1 to 20;
wherein n is an integer greater than 1.

10. The composition according to any preceding claim further comprising an initiator.

11. A method for preparing a composition according to any preceding claim, comprising steps of:
i) providing a pre-polymer having a polymeric backbone comprising a polymeric unit of the formula (-A-B-)ₙ, wherein A is derived from a substituted or unsubstituted polyol or mixture thereof and B is derived from a substituted or unsubstituted polyacid or mixture thereof, and n is an integer greater than 1;
ii) reacting the pre-polymer with an amine of formula NH₂-L-R to provide a pre-polymer comprising groups of formula (II): 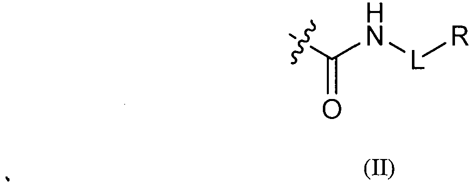 wherein L is a linking moiety, and wherein R is a moiety comprising a heteroatom
iii) activating the pre-polymer comprising the groups of formula (II) to provide an activated pre-polymer; and
iv)reacting the activated pre-polymer with an acid to provide a pre-polymer comprising groups of formula (I): 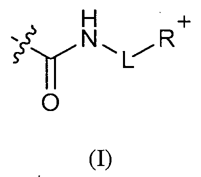 wherein R⁺ is a moiety comprising a positively charged heteroatom.

12. A method according to claim 11, wherein step (i) is a polymerization step wherein monomers are reacted to provide the pre-polymer backbone, wherein the monomers include a polyol, preferably a triol such as glycerol, and a diacid or a triacid, preferably sebacic acid.

13. A method according to claim 11 or claim 12, wherein the activation in step iii) is achieved by acrylation of hydroxy groups to give acrylate groups.

14. A method of curing a composition according any one of claims 1-10 or a composition obtainable by a method according to any one of claims 11-13, comprising a step of curing the composition with as stimulus, preferably with light in the presence of a photo-initiator.

15. A composition according to any one of claims 1-10 or a composition obtainable by a method according to any one of claims 11-13, for use in a method of fixing or sealing tissue, or for adhering tissue to the surface of a medical device.

16. A cured composition obtainable by the method of claim 14.

17. Use of a composition according to any one of claims 1-10 or a composition obtainable by a method according to any one of claims 11-13, in a method of fixing to or sealing tissue, or for fixing a medical device to the surface of a tissue.

18. Use of a composition according to any one of claims 1-10 or a composition obtainable by a method according to any one of claims 11-13, for preparing printing resin or for producing shaped objects by 3D printing.

19. Printed shaped objects prepared using a composition according to any one of claims 1-10 or a composition obtainable by a method according to any one of claims 11-13.
